# EUROPEAN PATENT SPECIFICATION

(11) **EP 0 321 967 B1**
(45) Date of publication and mention of the grant of the patent: **28.07.1993**
(21) Application number: 88121441.5
(22) Date of filing: 21.12.1988
(51) Int. Cl.: C07C 217/60, C07C 215/60, C07F 9/18, A61K 9/18, A61K 31/665

(54) **Compounds active on the cardiovascular system**
Kardiovaskulär aktive Verbindungen
Composés actifs sur le système cardiovasculaire

(30) Priority: 23.12.1987 IT 2318287
(43) Date of publication of application: 28.06.1989
(73) Proprietor: ZAMBON GROUP S.p.A., I-36100 Vicenza (IT)
(72) Inventor: Casagrande, Cesare, I-20020 Arese (MI) (IT); Norcini, Gabriele, I-21019 Maddalena Somma Lombardo (Va) (IT); Santangelo, Francesco, I-20148 Milan (IT)
(74) Representative: Weinhold, Peter, Dr.

(56) References cited:
- EP-A- 0 040 000
- EP-A- 0 062 596

## Description

The present invention relates to compounds active on the cardiovascular system and more particularly it relates to derivatives of N-propyl-dopamine and to their use in therapeutic field.

EP-A 0 062 596 discloses aminoethoxyphenyl which are useful therapeutical agents in the cardiovascular field.

Object of the present invention are the compounds of formula: 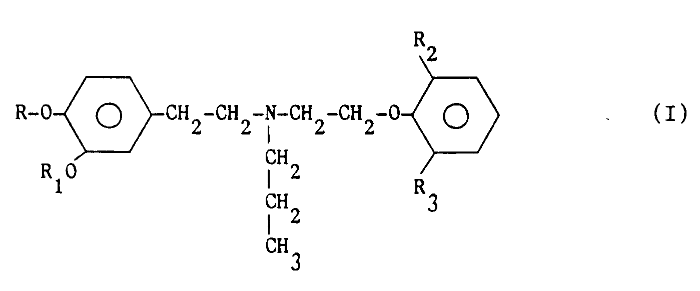
wherein
- R and R₁,: the same or different, represent hydrogen atoms or acyl groups derived from optionally substituted aliphatic, aromatic or heteroaromatic carboxylic acids, from optionally substituted carbamic or carbonic acids, or from phosphoric acid of formula 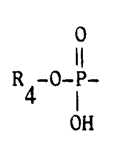 wherein
- R₄: represents a hydrogen atom, a C₁-C₆ alkyl optionally substituted by one or more groups selected from hydroxy, alkoxy, acyloxy, amino, carboxy and alkoxycarbonyl; or a phenyl; provided that when one of R and R₁ represents an acyl derived from phosphoric acid, the other represents a hydrogen atom;
- R₂ and R₃,: the same or different, represent a hydrogen or a halogen atom, an alkyl or an alkoxy group;
and their salts with pharmaceutically acceptable acids.

The compounds of formula I wherein R and R₁ represent hydrogen atoms are endowed with vasodilator and dopaminergic activity and they may be used in therapy in cardiovascular field as antihypertensive and for the treatment of cardiac insufficiency.

The compounds of formula I wherein one or both substituents R and R₁ are different from hydrogen are suitable pro-drugs of the active compounds and they may be used for the same therapeutic purposes.

The reason for the above proviso in the meanings of R and R₁ is in the fact that only the mono O-phosphate esters are suitable prodrugs of the active compounds.

If it is not otherwise specified, the terms alkyl or alkoxy in the compounds of formula I mean a linear or branched alkyl or alkoxy having from 1 to 4 carbon atoms, specific examples comprise methyl, ethyl, n.propyl, i.propyl, n.butyl, i.butyl, sec.butyl, ter.butyl, methoxy, ethoxy, n.propoxy, i.propoxy, ter.butoxy; preferred meanings are methyl and methoxy.

Halogen atoms comprise fluorine, chlorine, bromine or iodine, the first three being preferred.

The term acyl groups derived from aliphatic carboxylic acids means acyl radicals derived from linear or branched aliphatic carboxylic acids having from 1 to 6 carbon atoms, specific examples are the acyl groups derived from the following acids: formic, acetic, propionic, butyric, isobutyric, valeric and pivalic; acyl groups from aromatic or heteroaromatic carboxylic acids derive from optionally substituted benzoic or pyridinecarboxylic,(2-, 3- or 4-pyridinecarboxylic), pyrrolecarboxylic, isoxazolecarboxylic and quinolinecarboxylic acid. They may be substituted e.g. by alkyl or halogen. Specific examples comprise benzoyl, 2-pyridinecarbonyl, 3-pyridinecarbonyl, 4-pyridinecarbonyl, 2-chloro-benzoyl, 4-chloro-benzoyl, 2-methyl-benzoyl, 3-methyl-benzoyl, 4-methyl-benzoyl, 2,4-dimethyl-benzoyl, 4-nitro-benzoyl, 4-isobutyryl-benzoyl.

If R and R₁ are substituted aliphatic acids the preferred substituents are phenyl, one, two or three halogen atoms or alkoxy.

In case R and R₁ are substituted aromatic acids the preferred substituents are one, two or three halogen atoms, alkoxy, nitro, acyl groups derived from aliphatic carboxylic acids.

Preferred substituents for carbamic and carbonic acids are alkyl and phenyl.

Among the meanings of R₄, the term acyloxy means a acyloxy group having from 2 to 4 carbon atoms, a preferred example being acetoxy; the term alkoxycarbonyl means an alkoxycarbonyl group having from 1 to 4 carbon atoms in the alkoxy moiety, specific examples being methoxycarbonyl and ethoxycarbonyl.

Pharmaceutically acceptable acids useful for the preparation of salts with the compounds of formula I comprise hydrochloric, hydrobromic, hydriodic, nitric, sulfuric, phosphoric, acetic, benzoic, maleic, fumaric, succinic, tartaric, citric, aspartic and methanesulfonic acid.

Specific examples of compounds of formula I comprise:
N-propyl-N-(2-phenoxyethyl)dopamine
N-propyl-N-(2-phenoxyethyl)dopamine hydrochloride
N-propyl-N-(2-phenoxyethyl)dopamine succinate
N-propyl-N-[2-(2-methylphenoxy)-ethyl]-dopamine
N-propyl-N-[2-(2-chlorophenoxy)-ethyl]-dopamine
N-propyl-N-[2-(2-methoxyphenoxy)-ethyl]-dopamine
N-propyl-N-[2-(2,6-dimethylphenoxy)-ethyl]-dopamine
N-propyl-N-[2-(2,6-dimethoxyphenoxy)-ethyl]-dopamine
N-propyl-N-[2-(2,6-dichlorophenoxy)-ethyl]-dopamine
3,4-O-diisobutyryl-N-propyl-N-[2-(2-methoxyphenoxy)-ethyl]-dopamine
3,4-O-diacetyl-N-propyl-N-[2-(2,6-dichlorophenoxy)-ethyl]-dopamine
N-propyl-N-[2-(2-methoxyphenoxy)-ethyl]-dopamine-3-O-dihydrogenphosphate
N-propyl-N-[2-(2-methoxyphenoxy)-ethyl]-dopamine-3-O-methylhydrogenphosphate
N-propyl-N-[2-(2-bromophenoxy)-ethyl]-dopamine
N-propyl-N-[2-(2,6-diethylphenoxy)-ethyl]-dopamine
3,4-O-dipropionyl-N-propyl-N-(2-phenoxyethyl)-dopamine
3,4-O-di-(4-pyridinecarbonyl)-N-propyl-N-[2-(2,6-dichlorophenoxy)-ethyl]-dopamine
3,4-O-di-(4-chlorobenzoyl)-N-propyl-N-[2-(2,6-dimethoxyphenoxy)-ethyl]-dopamine
3,4-O-di-(dimethylcarbamoyl)-N-propyl-N-[2-(2,6-dichlorophenoxy)-ethyl]-dopamine
3,4-O-di-ethoxycarbonyl-N-propyl-N-[2-(2,6-dichlorophenoxy)-ethyl]-dopamine The preparation of the compounds of formula I can be carried out according to different alternative methods, which are described hereinafter.

Useful intermediates for some of these synthetic methods are N-propyl-dopamine or its derivatives (II) in which the aromatic hydroxy groups are protected, if desired 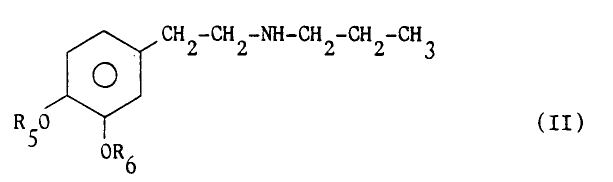
wherein
- R₅ and R₆,: the same or different, represent a hydrogen atom or a protective group selected, from methyl and benzyl.

Preferably R₅ and R₆ are the same when the aim of the synthesis is the preparation of compounds of formula I wherein R and R₁ are hydrogen atoms, but they may be different when the aim is the preparation of a compound of formula I wherein one of R and R₁ is different from hydrogen.

At the end of the synthetic process or before carrying out a reaction on an intermediate, the optional protective groups may be removed by conventional methods.

For example, when one or both R₅ and R₆ are methyl, the deprotection of the hydroxy group may be carried out by reaction with halogenidric acids or with boron tribromide: when one or both R₅ and R₆ are benzyl groups, the deprotection of the hydroxy group may be carried out by hydrogenolysis.

The compounds of formula II are useful starting compounds in three different synthetic methods:
a) the first method consists in a reaction between compound II and a halide of a suitable phenoxyacetic acid of formula 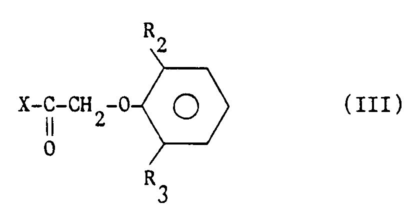 wherein
   R₂ and R₃ have the meanings reported for formula I and X represents a chlorine or a bromine atom.
   The reaction is carried out in an inert solvent in the presence of a base which acts as a halogenidric acid acceptor such as an alkaline carbonate or bicarbonate, a tertiary amine (triethylamine or pyridine). In the last case the amine may act also as a solvent.
   Through the above reaction the intermediates of formula 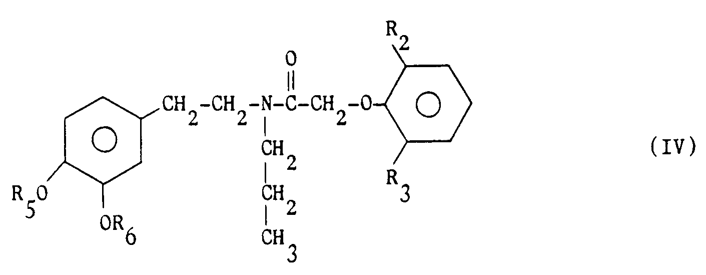 wherein
   R₂, R₃, R₅ and R₆ have the above reported meanings, are obtained.
   The reduction of the amidic carbonyl in the compounds of formula IV, preceded or followed by the optional deprotection of the hydroxy groups from the protective groups R₅ and R₆, gives the compounds of formula I wherein R and R₁ are hydrogen atoms.
   The reduction of the compounds of formula IV may be carried out with electrophilic reducing agents, particularly diborane optionally complexed with dimethylsulfide, tetrahydrofuran, aliphatic amines such as triethylamine or aromatic amines such as N,N-diethylaniline or pyridine.
   Alternatively, the reduction may be carried out with nucleophilic reducing agents such as metal hydrides, for example lithium aluminum hydride and sodium (2-methoxyethoxy)-aluminum hydride.
   The reaction is carried out in the presence of a solvent inert under the reaction conditions such as for example tetrahydrofuran, diethylether or 1,2-dimethoxyethane.
b) Alternatively, instead of the acyl halide III a phenoxyacetic aldehyde of formula 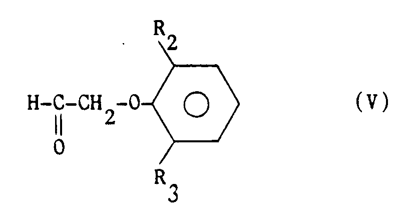 wherein R₂ and R₃ have the above reported meanings, may be used.
   By reaction with compound II, carried out in an inert solvent, optionally in the presence of dehydrating agents, and by a subsequent reduction, according to conventional methods, the compounds of formula I wherein R and R₁ are hydrogen atoms or their precursors, when R₅ and R₆ are different from hydrogen, are obtained.
   The above reduction is carried out in an inert solvent by catalytic hydrogenation or by reaction with reducing agents such as sodium borohydride, sodium cyanoborohydride and lithium aluminumhydride.
c) Another process for the preparation of the compounds of formula I consists in the condensation between a compound of formula II and a compound of formula 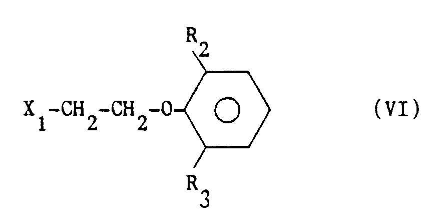 wherein R₂ and R₃ have the above reported meanings and X₁ represents a leaving group such as a chlorine or a bromine atom or an alkylsulfonyloxy or arylsulfonyloxy group.
   The reaction is carried out in an inert solvent and in the presence of a base and it gives directly the compounds of formula I wherein R and R₁ are hydrogen atoms or their precursors, when in the used compound of formula II R₅ and R₆ are different from hydrogen.

Further two alternative processes for the synthesis of the compounds of formula I, using intermediates different from N-propyl-dopamine or from its derivatives of formula II, are the following:
d) A process uses as starting product a compound of formula 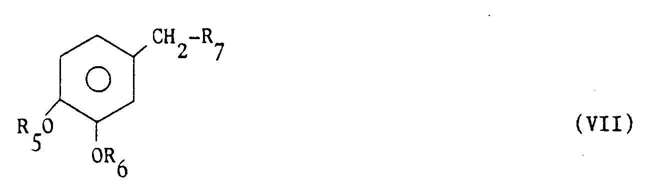 wherein
   - R₅ and R₆: have the above reported meanings and
   - R₇: represents a formyl group or a CH₂Y group wherein Y represents a halogen atom or an alkylsulfonyloxy or arylsulfonyloxy group;
   which is condensed with a secondary amine of formula 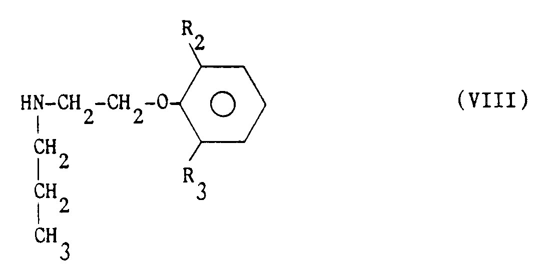 wherein R₂ and R₃ have the above reported meanings;
   by working in a way similar to what described in paragraph b. The compounds of formula I wherein R and R₁ are hydrogen atoms or their precursors when R₅ and R₆ are different from hydrogen are obtained.
e) Alternatively, dopamine or a derivative thereof of formula 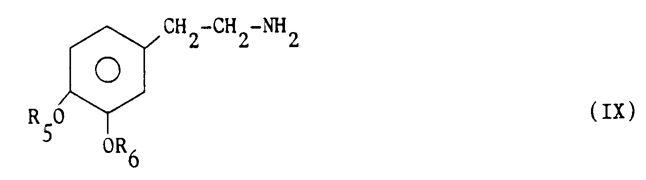 wherein R₅ and R₆ have the above reported meanings;
   may be used to obtain, following one of the synthetic methods described in paragraphs a, b or c, the intermediate of formula 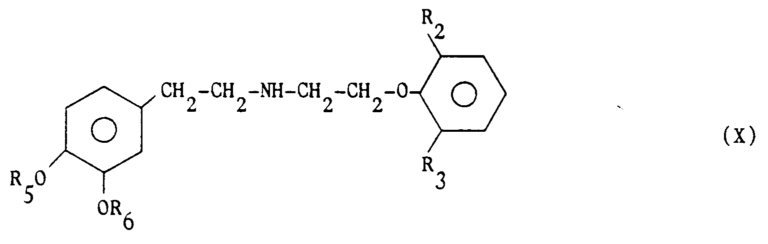 wherein R₂, R₃, R₅ and R₆ have the above reported meanings.
   This intermediate (X), by a subsequent alkylation with a propyl halide in a suitable organic solvent and optionally in the presence of a base, gives the compounds of formula I wherein R and R₁ are hydrogen atoms or their precursors wherein R₅ and R₆ are different from hydrogen.

The compounds of formula II, III, V, VI, VII, VIII and IX are known or they can be prepared by known methods.

The above described different synthetic methods are all substantially equivalent as far as their comparable yield and their versatility are concerned.

By working according to the above described methods the compounds of formula I in which R and R₁ represent hydrogen atoms or in which one or both of the aromatic hydroxy groups are in a protected form (for example as methylether or benzylether) are prepared.

The compounds of formula I in which both aromatic hydroxy groups are free (R=R₁=H) or one is protected (preferably in the form of benzylether) are also the starting compounds for the preparation of the compounds of formula I in which one or both R and R₁ are different from hydrogen.

The preparation of the compounds of formula I in which one or both R and R₁ are acyl groups derived from aliphatic or aromatic carboxylic acids is carried out by acylation according to conventional methods, for example with a suitable acyl halide or with an anhydride, or, when one of R and R₁ represents a group of formula 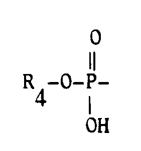
according to the method of monophosphorylation of catecholic hydroxy groups described in the European Patent Application No. 167204 in the name of Simes S.p.A. to which it is referred as far as the reagents and the experimental conditions are concerned.

When the compounds of formula I have one or more of the substituents R, R₁, R₂ and R₃ containing asymmetric carbon atoms, the compounds object of the present invention can exist in the form of stereoisomers.

The present invention relates to the possible stereoisomeric mixtures of the compounds of formula I as well as to the single stereoisomers obtained by optical separation or by stereospecific or stereoselective synthesis.

The preparation of the salts of the compounds of formula I with pharmaceutically acceptable acids is carried out according to usual methods.

A suitable method consists in adding the selected acid to a solution of compound I in a solvent from which the obtained salt precipitates and can be separated by filtration.

As above reported, the compounds of formula I are endowed with dopaminergic vasodilator activity and they may be used in therapy as antihypertensive and against cardiac insufficiency.

The compounds object of the present invention after administration by intravenous route in spontaneously hypertensive rat at the dose of 0.033-0.53 µm/kg induce a meaningful systemic arterial pressure decrease comprised between 20 and 90 mmHg.

In particular the compound N-[2-(2-methoxyphenoxy)ethyl]-N-propyl-dopamine hydrochloride (hereinafter referred to as compound A) administered at doses of 12.5 µg/kg and 200 µg/kg showed a reduction of systemic arterial pressure of 51 and 89 mmHg respectively in comparison with the basal value.

Furthermore, the dopaminergic activity as well as the α₁-antagonistic effect of the compounds object of the present invention has been evaluated.

The dopaminergic activity of compound A, for example, has been evaluated as percentage of receptor binding on isolated membranes of rat striated muscle; the IC₅₀ resulted to be 0.3 µM for D₂ receptor and 3 µM for D₁ receptor.

The α₁-antagonistic effect has been evaluated as antagonistic effect (pA₂) on vasoconstriction induced by phenylephrine (α₁-agonist) on rabbit isolated aorta. For compound A the obtained pA₂ value was 7.7.

For the practical administration, the compounds of formula I can be preferably prepared in the form of a pharmaceutical composition suitable for the selected administration route.

The pharmaceutical compositions containing the compounds of formula I, or their pharmaceutically acceptable salts, together with one or more solid or liquid, organic or inorganic pharmaceutical excipients and optionally further additives such as diluents, preserving agents, moistening agents, colouring agents and flavouring agents are a further object of the present invention.

The pharmaceutical compositions object of the invention can be administered in the form of solid pharmaceutical preparations, such as tablets, coated tablets, capsules, granulates and suppositories, or in the form of liquid pharmaceutical preparations such as syrups, suspensions, emulsions and solutions suitable for oral or parenteral administration.

The compounds object of the present invention can be formulated also in the form of slow and protracted release pharmaceutical preparations.

The preparation of the pharmaceutical compositions object of the present invention can be carried out according to usual techniques.

In order to better illustrate the present invention, the following examples are now given.

### Example 1

### Preparation of N-[2-(3,4-dihydroxyphenyl)-ethyl]-N-propyl-(2,6-dimethylphenoxy)-acetamide

To a solution of N-propyl-dopamine (5.52 g; 20 mmoles) and triethylamine (6.12 ml) in anhydrous dimethylformamide (100 ml), (2,6-dimethylphenoxy)-acetyl-chloride (3.97 g; 20 mmoles) in anhydrous dimethylformamide (10 ml) was added, at room temperature and under nitrogen.

The reaction mixture was kept under stirring for 3 hours and the solvent was evaporated under reduced pressure.

The obtained residue was dissolved in ethylacetate, washed with diluted HCl, then with water up to neutral pH.

After drying on sodium sulphate and evaporation of the solvent, the crude was dissolved in methanol.

To the so obtained solution p.toluenesulphonic acid (0.5 g) was added and the mixture was heated under reflux for 8 hours.

The solvent was evaporated and the residue was purified by column chromatography on silica gel (eluent methylene chloride:methanol = 97:3).

N-[2-(3,4-dihydroxyphenyl)-ethyl]-N-propyl-(2,6-dimethylphenoxy)-acetamide was obtained as a chromatographically pure oil (thin layer chromatography - eluent methylene chloride:methanol = 90:10).

¹H-NMR (60MHz-CDCl₃): delta (ppm): 0.8 (3H, t); 2.2 (3H, s); 2.3 3H, s); 4.0 (2H, s); 4.6 (2H, s); 6.8-7.0 (6H, m).

By working in a similar way the following compounds were prepared:

### N-[2-(3,4-dihydroxyphenyl)-ethyl]-N-propyl-(2,6-dimethoxyphenoxy)-acetamide

chromatographically pure oil (thin layer chromatography, eluent methylene chloride:methanol = 97:3)
¹H-NMR (60MHz-CDCl₃): delta (ppm): 0.9 (3H, s); 2.6 (2H, bt); 3.5 (4H, m); 3.9 (3H, s); 4.3 (1H, s); 4.6 (1H, s); 6.4-7.2 (6H, m).

### N-[2-(3,4-dihydroxyphenyl-ethyl]-N-propyl-(2,6-dichlorophenoxy)-acetamide

chromatographically pure oil (thin layer chromatography, eluent methylene chloride:methanol = 97:3)
¹H-NMR (60MHz-CDCl₃): delta (ppm): 0.9 (3H, s); 2.75 (2H, bt); 3.8 (4H, m); 4.5 (1H, s); 4.7 (1H, s); 6.4-7.2 (6H, m).

### N-[2-(3,4-dihydroxyphenyl)-ethyl]-N-propyl-phenoxyacetamide

chromatographically pure oil (thin layer chromatography, eluent methylene chloride:methanol = 90:10)
¹H-NMR (60MHz-CDCl₃): delta (ppm): 0.95 (3H, bs); 1.5 (2H, m); 2.8 (2H, bt); 3.5 (4H, m); 4.2 (1H, s). 4.7 (1H, s); 6.2-7.2 (8H, m).

### N-[2-(3,4-dihydroxyphenyl)-ethyl]-N-propyl-(2-chlorophenoxy)-acetamide

chromatographically pure oil (thin layer chromatography, eluent methylene chloride:methanol = 90:10)
¹H-NMR (60MHz-CDCl₃): delta (ppm): 0.8 (3H, t); 1.5 (2H, m); 2.9 (2H, t); 3.5 (4H, m); 4.3 (1H, s); 4.8 (1H, s); 6.4-8.4 (7H, m).

### N-[2-(3,4-dihydroxyphenyl)-ethyl]-N-propyl-(2-methylphenoxy)-acetamide

chromatographically pure oil (thin layer chromatography, eluent methylene chloride:methanol = 90:10)
¹H-NMR (60MHz-CDCl₃): delta (ppm): 0.8 (3H, t); 2.6 (2H, m); 2.2 3H, d); 2.7 (2H, bt); 3.5 (4H, m); 4.2 (1H, s); 4.7 (1H, s); 6.4-8.4 (7H, m).

### N-[2-(3,4-dihydroxyphenyl)-ethyl]-N-propyl-(2-methoxyphenoxy)-acetamide

chromatographically pure oil (thin layer chromatography, eluent methylene chloride:methanol = 90:10)
¹H-NMR (60MHz-CDCl₃): delta (ppm): 0.9 (3H, s); 2.7 (2H, bt); 3.8 (3H, s); 3.85 (3H, s); 4.5 (1H, s); 4.6 (1H, s); 6.4-7.2 (6H, m).

### Example 2

### Preparation of N-[2-(2,6-dimethylphenoxy)-ethyl]-N-propyl-dopamine hydrochloride

To a solution of N-[2-(3,4-dihydroxyphenyl)-ethyl]-N-propyl-(2,6-dimethylphenoxy)-acetamide (5.5 g; 15.4 mmoles), prepared as described in example 1, in tetrahydrofuran (55 ml), under nitrogen and keeping the temperature at 0-5°C, BH₃.S(CH₃)₂ (5.583 ml; 61.5 mmoles) was added.

The reaction mixture was heated under reflux for 30 minutes and, after cooling at 5-10°C, HCl 6N (55 ml) was added and the mixture was heated under reflux for 30 minutes.

The solvent was evaporated and the residue was dissolved in water, neutralized with sodium carbonate and extracted with methylene chloride.

After drying on sodium sulphate and evaporation of the solvent a crude, which was dissolved in ethanol, was obtained.

From the solution, acidified with ethyl ether saturated by HCl, N-[2-(2,6-dimethylphenoxy)-ethyl]-N-propyl-dopamine crystallized as hydrochloride with m.p. 104-105°C (ethanol/ethyl ether).

By working in a similar way the following compounds were prepared:

### N-[2-(2,6-diemthylphenoxy)-ethyl]-N-propyl-dopamine hydrochloride

m.p.=107-109°C (ethanol/ethyl ether)

### N-[2-(2,6-dichlorophenoxy)-ethyl]-N-propyl-dopamine hydrochloride

m.p.=126-129°C (ethanol/ethyl ether)

### N-(2-phenoxyethyl)-N-propyl-dopamine hydrochloride

m.p.=137-138°C (ethanol/ethyl ether)

### N-[2-(2-chlorophenoxy)-ethyl]-N-propyl-dopamine hydrochloride

m.p.=124-126°C (ethanol/ethyl ether)

### N-[2-(2-methylphenoxy)-ethyl]-N-propyl-dopamine hydrochloride

m.p.=141-143°C (ethanol/ethyl ether)

### N-[2-(2-methoxyphenoxy)-ethyl]-N-propyl-dopamine hydrochloride

m.p.=187-188°C (ethanol/ethyl ether)

### Example 3

### Preparation of 3,4-O-di-isobutyryl-N-[2-(2-methoxyphenoxy)-ethyl]-N-propyl-dopamine maleate

To a solution of N-[2-(2-methoxyphenoxy)-ethyl]-N-propyl-dopamine hydrochloride (4 g; 11.7 mmoles), prepared as described in example 2, in pyridine (30 ml) isobutyrylchloride (4 ml) was added.

After 4 hours at room temperature, the reaction mixture was poured into water and extracted with methylene chloride.

The organic phase was evaporated to residue, treated with ethyl ether, washed with a sodium bicarbonate diluted solution and dried on sodium sulphate.

After evaporation of the solvent an oil was obtained which was dissolved in ethyl ether.

By acidification of the solution with an ethanolic solution saturated with maleic acid, 3,4-O-di-isobutyryl-N-[2-(2-methoxyphenoxy)-ethyl]-N-propyl-dopamine maleate was separated m.p. 100-102°C (ethyl acetate).

## Claims (Claims for the following Contracting State(s): AT, BE, CH, DE, FR, GB, GR, IT, LI, LU, NL, SE)

1. A compound of formula 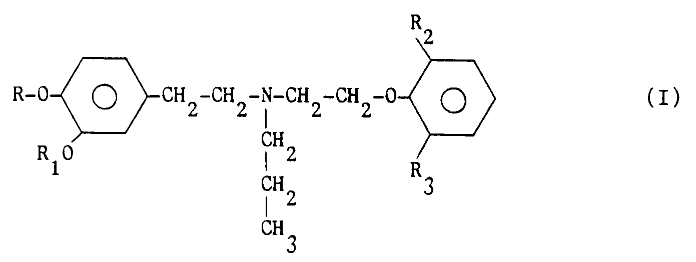 wherein
R and R₁, the same or different, represent hydrogen atoms or acyl groups derived from optionally substituted aliphatic, aromatic or heteroaromatic carboxylic acids, from optionally substituted carbamic or carbonic acids, or from phosphoric acid of formula 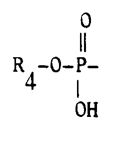 wherein
R₄ represents a hydrogen atom, a C₁-C₆ alkyl optionally substituted by one or more groups selected from hydroxy, alkoxy, acyloxy, amino, carboxy and alkoxycarbonyl, or a phenyl; provided that when one of R and R₁ represents an acyl derived from phosphoric acid, the other represents a hydrogen atom;
R₂ and R₃, the same or different, represent a hydrogen or a halogen atom, an alkyl or an alkoxy group;
and their salts with pharmaceutically acceptable acids.

2. A compound, according to claim 1, wherein R and R₁ the same or different, represent acyl groups derived from carboxylic acids selected from formic, acetic, propionic, butyric, isobutyric, valeric, pivalic, optionally substituted benzoic, or optionally substituted 2-pyridinecarboxylic, 3-pyridinecarboxylic, 4-pyridinecarboxylic acid.

3. A compound, according to claim 1, wherein one of R and R₁ represents a hydrogen atom and the other represents an acyl group derived from phosphoric acid of formula 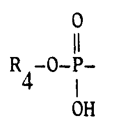 wherein
R₄ represents a hydrogen atom or a methyl or an ethyl group optionally substituted by one or more groups selected from hydroxy, methoxy, acetoxy, amino, carboxy, methoxycarbonyl, ethoxycarbonyl and phenyl.

4. A compound, according to claim 1, wherein R and R₁ represent hydrogen atoms.

5. A process for the preparation of the compounds according to claim 1 comprising:
a) the reaction between N-propyl-dopamine or a derivative thereof of formula 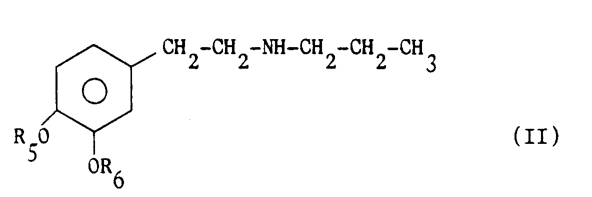 wherein
R₅ and R₆, the same or different, represent a hydrogen atom or a protective group selected from methyl and benzyl;
and a halide of a suitable phenoxyacetic acid of formula 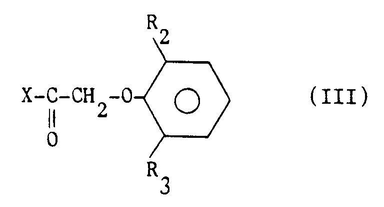 wherein
R₂ and R₃, the same or different, represent a hydrogen or a halogen atom, an alkyl or an alkoxy group and X represents a chlorine or a bromine atom;
in an inert solvent in the presence of a base, which acts as a halogenidric acid acceptor, selected from an alkaline carbonate or bicarbonate or a tertiary amine selected from triethylamine or pyridine.
b) the reduction of the so obtained intermediate of formula 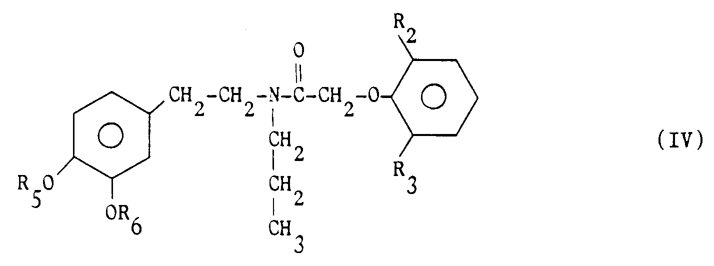 wherein
R₂, R₃, R₅ and R₆ have the above reported meanings; with electrophilic or nucleophilic reducing agents selected from diborane, optionally complexed with dimethylsulfide, tetrahydrofuran and aliphatic or aromatic amine, lithium aluminum hydride and sodium (2-methoxyethyl)-aluminum hydride in a solvent inert under the reaction conditions.

6. A pharmaceutical composition containing a therapeutically effective amount of a compound according to claim 1 together with one or more pharmaceutically acceptable excipients.

## Claims (Claims for the following Contracting State(s): ES)

1. A process for the preparation of the compounds of formula 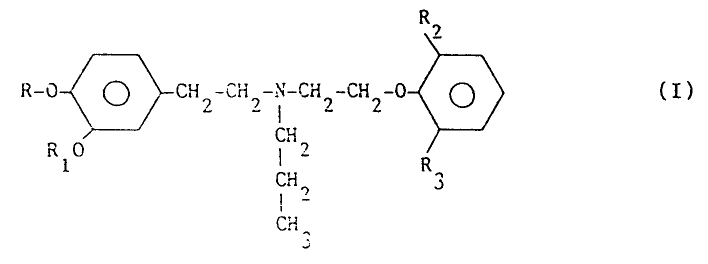 wherein
R and R₁, the same or different, represent hydrogen atoms or acyl groups derived from optionally substituted aliphatic, aromatic or heteroaromatic carboxylic acids, from optionally substituted carbamic or carbonic acids, or from phosphoric acid of formula 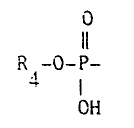 wherein
R₄ represents a hydrogen atom, a C₁-C₆ alkyl optionally substituted by one or more groups selected from hydroxy, alkoxy, acyloxy, amino, carboxy and alkoxycarbonyl, or a phenyl; provided that when one of R and R₁ represents an acyl derived from phosphoric acid, the other represents a hydrogen atom;
R₂ and R₃, the same or different, represent a hydrogen or a halogen atom, an alkyl or an alkoxy group;
and their salts with pharmaceutically acceptable acids comprising
a) the reaction between N-propyl-dopamine or a derivative thereof of formula 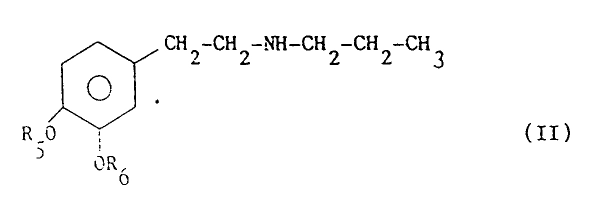 wherein
R₅ and R₆, the same or different, represent a hydrogen atom or a protective group selected from methyl and benzyl;
and a halide of a phenoxyacetic acid of formula 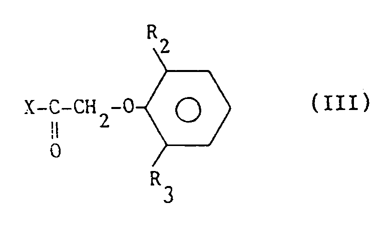 wherein
R₂ and R₃, the same or different, represent a hydrogen or a halogen atom, an alkyl or an alkoxy group and X represents a chlorine or a bromine atom;
in an inert solvent in the presence of a base, which acts as a halogenidric acid acceptor, selected from an alkaline carbonate or bicarbonate or a tertiary amine selected from triethylamine or pyridine.
b) the reduction of the so obtained intermediate of formula 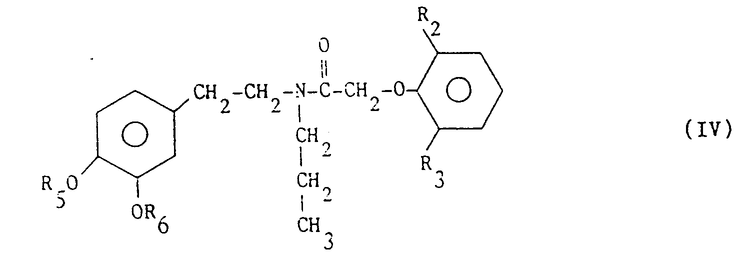 wherein
R₂, R₃, R₅ and R₆ have the above reported meanings; with electrophilic or nucleophilic reducing agents selected from diborane, optionally complexed with dimethylsulfide, tetrahydrofuran and aliphatic or aromatic amine, lithium aluminum hydride and sodium (2-methoxyethoxy)-aluminum hydride in a solvent inert under the reaction conditions.

## Patentansprüche (Patentansprüche für folgende(n) Vertragsstaat(en): AT, BE, CH, DE, FR, GB, GR, IT, LI, LU, NL, SE)

1. Verbindung der Formel 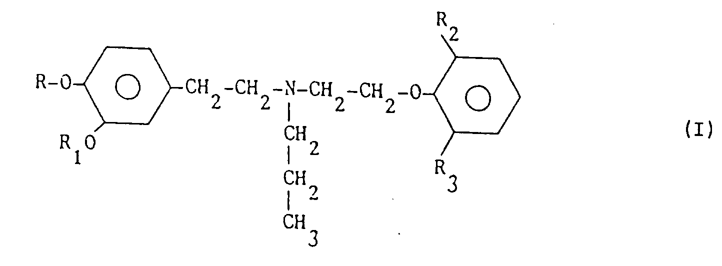 worin
R und R₁, die gleich oder verschieden sein können, Wasserstoffatome bedeuten oder Acylgruppen, die abgeleitet sind von wahlweise substituierten aliphatischen, aromatischen oder heteroaromatischen Carbonsäuren, von wahlweise substituierten Carbamin- oder Kohlensäuren, oder von Phosphorsäure der Formel 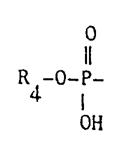 worin
R₄ ein Wasserstoffatom, eine wahlweise mit ein oder mehreren Gruppen, die ausgewählt sind aus Hydroxy, Alkoxy, Acyloxy, Amino, Carboxy und Alkoxycarbonyl, substituierte C₁-C₆-Alkylgruppe, oder eine Phenylgruppe bedeutet, unter der Bedingung, daß, wenn eine der Gruppen R und R₁ eine von Phosphorsäure abgeleitete Acylgruppe darstellt, die andere Gruppe ein Wasserstoffatom bedeutet;
R₂ und R₃, die gleich oder verschieden sein können, ein Wasserstoff- oder ein Halogenatom, eine Alkyl- oder eine Alkoxygruppe bedeuten;
und ihre Salze mit pharmazeutisch annehmbaren Säuren.

2. Verbindung nach Anspruch 1, worin R und R₁, die gleich oder verschieden sein können, Acylgruppen bedeuten, die sich von Carbonsäuren ableiten, die ausgewählt sind aus der Gruppe Ameisensäure, Essigsäure, Propionsäure, Buttersäure, Isobuttersäure, Valeriansäure, Pivalinsäure, wahlweise substituierter Benzoesäure oder wahlweise substituierter 2-Picolinsäure, 3-Picolinsäure und 4-Picolinsäure.

3. Verbindung nach Anspruch 1, worin eine der Gruppen R und R₁ ein Wasserstofatom bedeutet und die andere eine Acylgruppe, abgeleitet von Phosphorsäure der Formel 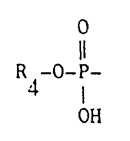 worin
R₄ ein Wasserstoffatom oder eine Methyl- oder Ethylgruppe bedeutet, wahlweise substituiert mit ein oder mehreren Gruppen, die ausgewählt sind aus Hydroxy, Methoxy, Acetoxy, Amino, Carboxy, Methoxycarbonyl, Ethoxycarbonyl und Phenyl.

4. Verbindung nach Anspruch 1, worin R und R₁ Wasserstoffatome bedeuten.

5. Verfahren zur Herstellung von Verbindungen nach Anspruch 1, das umfaßt:
a) die Reaktion zwischen N-Propyldopamin oder einem seiner Derivate der Formel 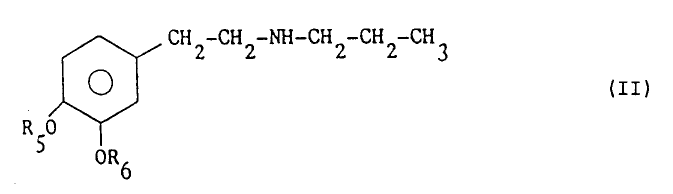 worin
R₅ und R₆, die gleich oder verschieden sein können, ein Wasserstoffatom oder eine Schutzgruppe, ausgewählt aus Methyl und Benzyl, bedeuten;
und einem Halogenid einer Phenoxyessigsäure der Formel 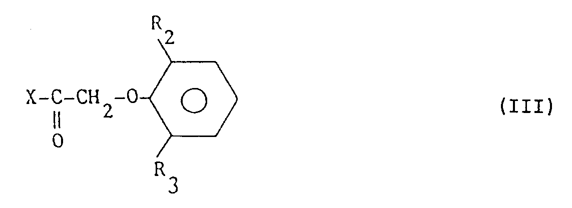 worin
R₂ und R₃, die gleich oder verschieden sein können, ein Wasserstoffatom oder ein Halogenatom, eine Alkyl-oder eine Alkoxygruppe bedeuten und X ein Chlor- oder Bromatom bedeutet;
in einem inerten Lösungsmittel in Gegenwart einer Base, die als Akzeptor für Halogenwasserstoffsäure fungiert und ausgewählt ist aus einem Alkalicarbonat oder -bicarbonat oder einem tertiären Amin, ausgewählt aus Triethylamin oder Pyridin.
b) die Reduktion des so erhaltenen Zwischenprodukts der Formel 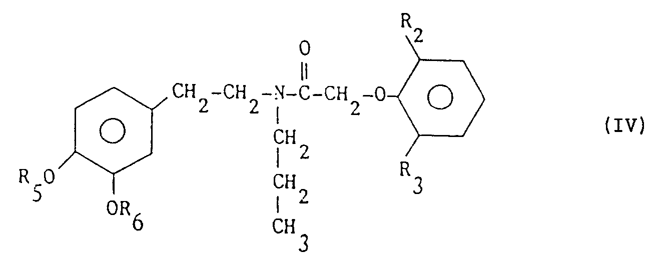 worin
R₂, R₃, R₅ und R₆ die obige Bedeutung haben; mit elektrophilen oder nucleophilen Reduktionsmitteln, ausgewählt aus Diboran, wahlweise komplexiert mit Dimethylsulfid, Tetrahydrofuran und aliphatischen oder aromatischen Aminen, Lithiumaluminiumhydrid und Natrium-(2-methoxyethoxy)-aluminiumhydrid, in einem unter den Reaktionsbedingungen inerten Lösungsmittel.

6. Pharmazeutische Zusammensetzung, die eine therapeutisch wirksame Menge einer Verbindung nach Anspruch 1 zusammen mit ein oder mehreren pharmazeutisch annehmbaren Bindemitteln enthält.

## Patentansprüche (Patentansprüche für folgende(n) Vertragsstaat(en): ES)

1. Verfahren zur Herstellung der Verbindungen der Formel 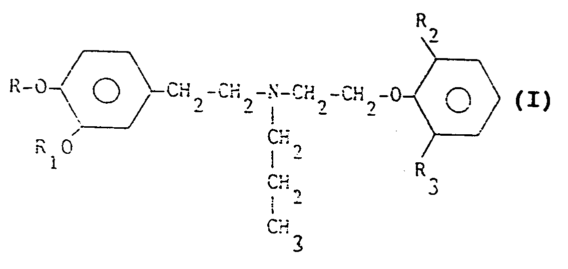 worin
R und R₁, die gleich oder verschieden sein können, Wasserstoffatome bedeuten oder Acylgruppen, die abgeleitet sind von wahlweise substituierten aliphatischen, aromatischen oder heteroaromatischen Carbonsäuren, von wahlweise substituierten Carbamin- oder Kohlensäuren oder von Phosphorsäure der Formel 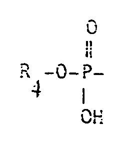 worin
R₄ ein Wasserstoffatom, eine wahlweise mit ein oder mehreren Gruppen, die ausgewählt sind aus Hydroxy, Alkoxy, Acyloxy, Amino, Carboxy und Alkoxycarbonyl, substituierte C₁-C₆-Alkylgruppe oder eine Phenylgruppe bedeutet, unter der Bedingung, daß, wenn eine der Gruppen R und R₁ eine von Phosphorsäure abgeleitete Acylgruppe darstellt, die andere Gruppe ein Wasserstoffatom bedeutet;
R₂ und R₃, die gleich oder verschieden sein können, ein Wasserstoff- oder ein Halogenatom, eine Alkyl- oder eine Alkoxygruppe bedeuten;
und ihrer Salze mit pharmazeutisch annehmbaren Säuren, welches umfaßt:
a) die Reaktion zwischen N-Propyldopamin oder einem seiner Derivate der Formel 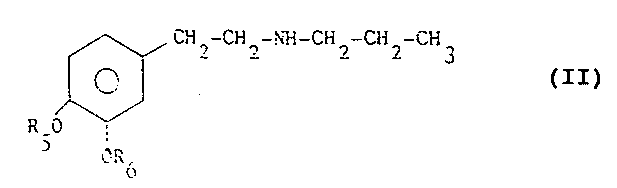 worin
R₅ und R₆, die gleich oder verschieden sein können, ein Wasserstoffatom oder eine Schutzgruppe, ausgewählt aus Methyl und Benzyl bedeuten;
und einem Halogenid einer Phenoxyessigsäure der Formel 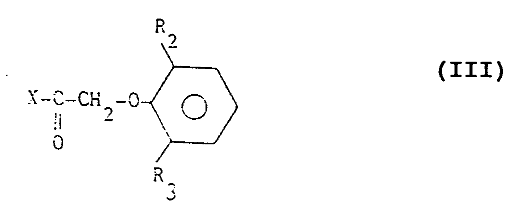 worin
R₂ und R₃, die gleich oder verschieden sein können, ein Wasserstoffatom oder ein Halogenatom, eine Alkyl- oder eine Alkoxygruppe bedeuten und X ein Chlor- oder Bromatom bedeutet;
in einem inerten Lösungsmittel in Gegenwart einer Base, die als Akzeptor für Halogenwasserstoffsäure fungiert und ausgewählt ist aus einem Alkalicarbonat oder -bicarbonat oder einem tertiären Amin, ausgewählt aus Triethylamin oder Pyridin;
b) die Reduktion des so erhaltenen Zwischenprodukts der Formel 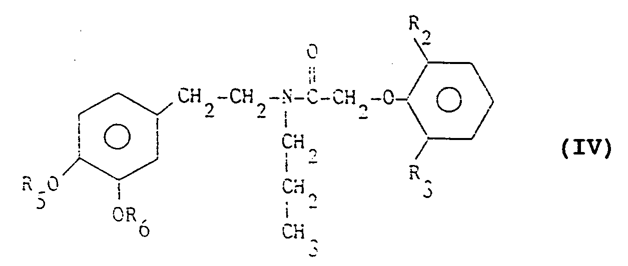 worin
R₂, R₃, R₅ und R₆ die oben definierten Bedeutungen haben;
mit elektrophilen oder nucleophilen Reduktionsmitteln, ausgewählt aus Diboran, wahlweise komplexiert mit Dimethylsulfid, Tetrahydrofuran und aliphatischen oder aromatischen Aminen, Lithiumaluminiumhydrid und Natrium-(2-methoxyethoxy)-aluminiumhydrid, in einem unter den Reaktionsbedingungen inerten Lösungsmittel.

## Revendications (Revendications pour l'(les) Etat(s) contractant(s) suivant(s): AT, BE, CH, DE, FR, GB, GR, IT, LI, LU, NL, SE)

1. Composé de formule 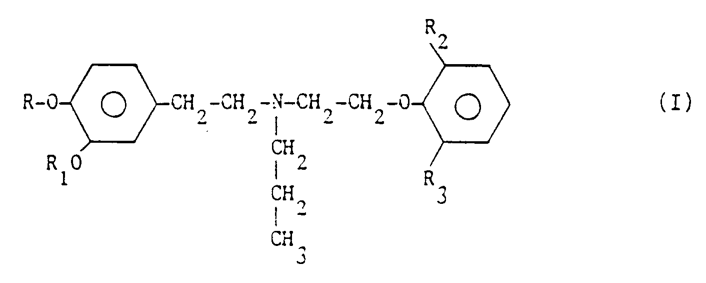 dans laquelle
R et R₁, qui peuvent être identiques ou différents, représentent des atomes d'hydrogène ou des groupements acyle dérivés d'acides carboxyliques aliphatiques, aromatiques ou hétéroaromatiques éventuellement substitues, d'acides carbamiques ou carboniques éventuellement substitués ou d'acide phosphorique de formule 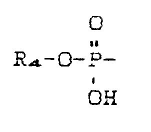 où
R₄ représente un atome d'hydrogène, un reste alkyle en C₁-C₆ éventuellement substitué par un ou plusieurs groupements choisis parmi les groupements hydroxy, alcoxy, acyloxy, amino, carboxy et alcoxycarbonyle, ou un reste phényle;
étant spécifié que quand l'un des restes R et R₁ représente un groupement acyle dérivé d'acide phosphorique, l'autre représente un atome d'hydrogène;
R₂ et R₃, qui peuvent être identiques ou différents, représentent chacun un atome d'hydrogène ou d'halogène ou un groupement alkyle ou alcoxy;
et ses sels avec des acides acceptables pharmaceutiquement.

2. Composé selon la revendication 1, dans lequel R et R₁, qui peuvent être identiques ou différents, représentent des groupements acyle dérivés d'acides carboxyliques choisis parmi les acides formique, acétique, propionique, butyrique, isobutyrique, valérique, pivalique, benzoïque éventuellement substitué ou 2-pyridinecarboxylique, 3-pyridinecarboxylique ou 4-pyridinecarboxylque éventuellement substitué.

3. Composé selon la revendication 1, dans lequel l'un des restes R et R₁ représente un atome d'hydrogène et l'autre représente un groupement acyle dérivé d'acide phosphorique de formule 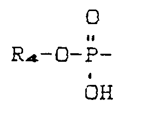 dans laquelle
R₄ représente un atome d'hydrogène, ou un groupement méthyle ou éthyle éventuellement substitué par un ou plusieurs groupements choisis parmi les groupements hydroxy, methoxy, acetoxy, amino, carboxy, méthoxycarbonyle, éthoxycarbonyle et phényle.

4. Composé selon la revendication 1, dans lequel R et R₁ représentent des atomes d'hydrogène.

5. Procédé de préparation des composés selon la revendication 1, comprenant:
a) la réaction entre de la N-propyldopamine ou un dérivé de celle-ci de formule 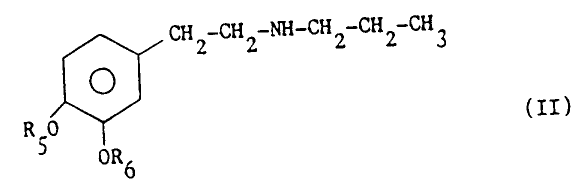 dans laquelle
R₅ et R₆, qui peuvent être identiques ou différents, représentent chacun un atome d'hydrogène ou un groupement protecteur choisi entre les groupements méthyle et benzyle,
et un halogénure d'un acide phénoxyacétique de formule 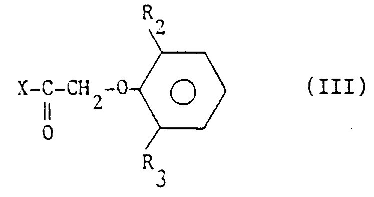 dans laquelle
R₂ et R₃, qui peuvent être identiques ou différents, représentent chacun un atome d'hydrogène ou d'halogène ou un groupement alkyle ou alcoxy et
X représente un atome de chlore ou de brome,
dans un solvant inerte, en présence d'une base qui sert d'accepteur d'hydracide halogéné, choisie parmi un carbonate ou bicarbonate alcalin et une amine tertiaire choisie entre la triéthylamine et la pyridine;
b) la réduction du produit intermédiaire ainsi obtenu, de formule 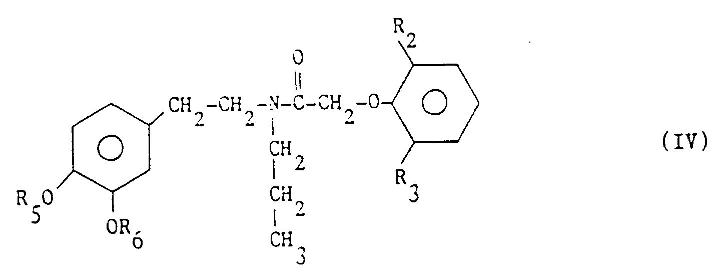 dans laquelle
R₂, R₃, R₅ et R₆ ont les significations sus-indiquées,
par des agents de réduction électrophiles ou nucléophiles choisis parmi le diborane, éventuellement complexé avec du sulfure de diméthyle, du tétrahydrofuranne et une amine aliphatique ou aromatique, l'hydrure de lithium et d'aluminium et l'hydrure de sodium et de (2-méthoxyéthoxy)aluminium, dans un solvant inerte dans les conditions de la réaction.

6. Composition pharmaceutique, contenant une quantité thérapeutiquement efficace d'un composé selon la revendication 1, en combinaison avec un ou plusieurs excipients acceptable pharmaceutiquement.

## Revendications (Revendications pour l'(les) Etat(s) contractant(s) suivant(s): ES)

1. Procédé de préparation des composés de formule 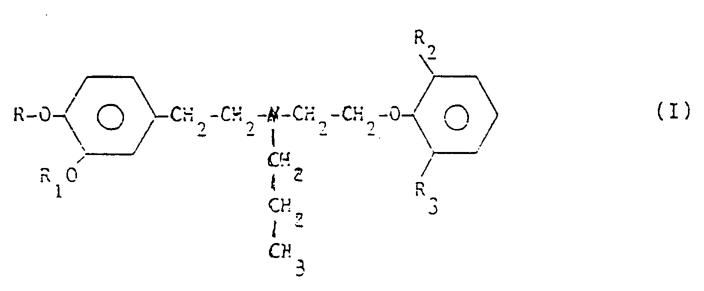 dans laquelle
R et R₁, qui peuvent être identiques ou différents, représentent des atomes d' hydrogène ou des groupements acyle dérivés d'acides carboxyliques aliphatiques, aromatiques ou hétéroaromatiques éventuellement substitués, d'acides carbamiques ou carboniques éventuellement substitués ou d'acide phosphorique de formule 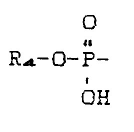 où
R₄ représente un atome d'hydrogène, un reste alkyle en C₁-C₆ éventuellement substitué par un ou plusieurs groupements choisis parmi les groupements hydroxy, alcoxy, acyloxy, amino, carboxy et alcoxycarbonyle, ou un reste phényle;
étant spécifié que quand l'un des restes R et R₁ représente un groupement acyle dérivé d'acide phosphorique, l'autre représente un atome d'hydrogène;
R₂ et R₃, qui peuvent être identiques ou différents, représentent chacun un atome d'hydrogène ou d'halogène ou un groupement alkyle ou alcoxy;
et de leurs sels avec des acides acceptables pharmaceutiquement,
comprenant:
a) la réaction entre de la N-propyldopamine ou un dérivé de celle-ci de formule 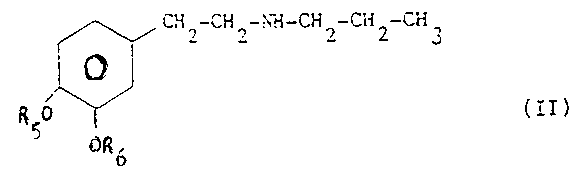 dans laquelle
R₅ et R₆, qui peuvent être identiques ou différents, représentent chacun un atome d'hydrogène ou un groupement protecteur choisi entre les groupements méthyle et benzyle,
et un halogénure d'un acide phénoxyacétique de formule 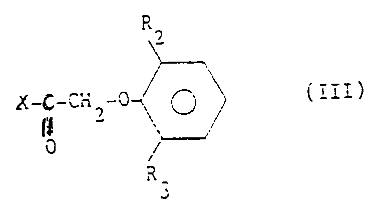 dans laquelle
R₂ et R₃, qui peuvent être identiques ou différents, représentent chacun un atome d'hydrogène ou d'halogène ou un groupement alkyle ou alcoxy et
X représente un atome de chlore ou de brome,
dans un solvant inerte, en présence d'une base qui sert d'accepteur d'hydracide halogéné, choisie parmi un carbonate ou bicarbonate alcalin et une amine tertiaire choisie entre la triéthylamine et la pyridine;
b) la réduction du produit intermédiaire ainsi obtenu, de formule 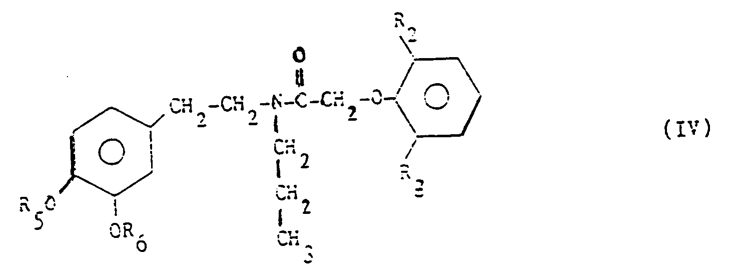 dans laquelle
R₂, R₃, R₅ et R₆ ont les significations sus-indiquées,
par des agents de réduction électrophiles ou nucléophiles choisis parmi le diborane, éventuellement complexé avec du sulfure de diméthyle, du tétrahydrofuranne et une amine aliphatique ou aromatique, l'hydrure de lithium et d'aluminium et l'hydrure de sodium et de (2-méthoxyéthoxy)aluminium, dans un solvant inerte dans les conditions de la réaction.
